# EUROPÄISCHE PATENTSCHRIFT

(11) **EP 2 471 688 B1**
(45) Veröffentlichungstag und Bekanntmachung des Hinweises auf die Patenterteilung: **25.02.2015**
(21) Anmeldenummer: 11195987.0
(22) Anmeldetag: 29.12.2011
(51) Int. Cl.: B60Q 1/32, B60Q 1/26

(54) **Außenliegendes Montagebauteil für ein Fahrzeug mit einem Lichtmodul**
External assembly component for a vehicle with a light module
Composant de montage extérieur pour un véhicule doté d'un module d'éclairage

(30) Priorität: 30.12.2010 DE 102010061643
(43) Veröffentlichungstag der Anmeldung: 04.07.2012
(73) Patentinhaber: Huf Hülsbeck & Fürst GmbH & Co. KG, 42551 Velbert (DE)
(72) Erfinder: Lennhoff, Ralf, 58093 Hagen (DE)
(74) Vertreter: Bals, Rüdiger

(56) Entgegenhaltungen:
- EP-A1- 2 179 890
- WO-A1-2008/137634

## Beschreibung

Die Erfindung betrifft ein außenliegendes Montagebauteil für ein Fahrzeug, insbesondere in Form eines Außenspiegels, Türgriffs, Türschweller, Schürze oder dergleichen, mit einem Lichtmodul, welches zumindest ein nach außen, sichtbares Licht emittierendes Leuchtmittel aufweist, gemäß dem Oberbegriff von Anspruch 1. Ferner betrifft die Erfindung auch ein Fertigungsverfahren für ein Montagebauteil mit einem Lichtmodul gemäß dem Oberbegriff von Anspruch 15.

Derartige Montagemodule sind im Bereich der Kraftfahrzeugtechnik überall dort bekannt, wo beispielsweise Licht erzeugt und abgestrahlt werden soll. Beispielsweise seien Scheinwerfer, Rücklichter, Bremslichter, Blinker und dergleichen genannt. Um den Komfort bei Fahrzeugen zu erhöhen, wird eine so genannte Vorfeldbeleuchtung bei eintretender Dunkelheit benutzt, die das Auffinden des Fahrzeuges und den Einstieg ins Fahrzeug erleichtern sollen. Dabei können auch die Türgriffe, die zum Einstieg ins Fahrzeug betätigt werden müssen, zusätzlich beleuchtet werden, indem zum Beispiel die Mulde unter dem Türgriff ausgeleuchtet wird. Das Einschalten einer derartigen Beleuchtung kann zum Beispiel durch eine Annäherung einer Person an das Fahrzeug erfolgen. Hierbei kann es notwendig sein, dass die entsprechende Person mit dem richtigen ID-Geber ausgestattet ist, damit die Vorfeldbeleuchtung oder Rundumbeleuchtung des Fahrzeuges bei Dunkelheit eingeschaltet wird. Ebenfalls kann das Einschalten der Beleuchtung auch durch einen aktiven Vorgang, wie zum Beispiel ein Tastendruck auf den per Funk wirkenden ID-Geber ausgelöst werden, der zur Ansteuerung des Zugangskontrollsystems genutzt wird.

Aus der Druckschrift WO 2008 / 137634 A1 ist beispielsweise ein gattungsgemäßes Lichtmodul für einen Türgriff bekannt, das eine Muldenbeleuchtung und eine Vorfeldbeleuchtung ermöglicht. Dieses Lichtmodul ist über flexible elektrische Leitungen mit einer Steckerbuchse verbunden, die dazu dient, das Lichtmodul mit der fahrzeugseitigen Elektronik zu verbinden. Nachteilig hat sich bei diesem Türaußengriff herausgestellt, dass die Fertigung des Türgriffes mit dem Lichtmodul aufwändig ist, da dieses aus zahlreichen Einzelteilen besteht, die mit dem Türgriff dann verbunden werden müssen. Außerdem ist die Montage des Türaußengriffes mit dem entsprechenden Lichtmodul am Fahrzeug ebenfalls schwierig, da zunächst der an den flexiblen elektrischen Anschlussleitungen hängende Stecker des Lichtmoduls durch ein Blechöffnung der Fahrzeugtür eingefädelt werden muss, um dann den Türgriff mit seinem Lagerarm durch dieselbe Öffnung einzuschieben. Anschließend muss der entsprechende Stecker des Lichtmoduls von der Innenseite der Fahrzeugtür mit dem fahrzeugseitigen Gegenstecker verbunden werden, wozu ein weiterer Montageschritt notwendig ist.

Aufgabe der vorliegenden Erfindung ist es, ein außenliegendes Montagebauteil für ein Fahrzeug mit einem Lichtmodul bereitzustellen, das einerseits einfach hergestellt werden kann und andererseits einfach am Fahrzeug montiert werden kann.

Die vorliegende Aufgabe wird durch ein außenliegendes Montagebauteil mit den Merkmalen des Anspruches 1, insbesondere aus dem kennzeichnenden Teil, gelöst. Ebenfalls wird zur Lösung der Aufgabe ein Fertigungsverfahren mit den Merkmalen des Anspruches 15 vorgeschlagen. In den abhängigen Vorrichtungs- und Verfahrensansprüchen sind bevorzugte Weiterbildungen der Erfindung ausgeführt. Merkmale die zu dem erfindungsgemäßen Montagebauteil offenbart werden gelten auch für das erfindungsgemäße Montageverfahren und umgekehrt. Außerdem kann das erfindungsgemäße Verfahren aus Anspruch 15 für das erfindungsgemäße Montagebauteil durchgeführt werden.

Erfindungsgemäß ist es bei dem Montagebauteil vorgesehen, dass das Lichtmodul ein separates Bauteil zum Montagebauteil darstellt, welches an dem Montagebauteil befestigt ist und der Stecker einteilig mit dem Lichtmodul, insbesondere dem Gehäuse des Lichtmoduls, ausgestaltet ist. Hierbei ist der Stecker insbesondere materialeinheitlich und einstückig zum Gehäuse des Lichtmoduls ausgestaltet. Da der Stecker nicht wie aus dem Stand der Technik bekannt, über eine flexible elektrische Anschlussleitung mit dem Lichtmodul verbunden ist, entfällt diese elektrische Leitung. Somit lässt sich das Lichtmodul auch kostengünstiger fertigen und leichter an dem außenliegenden Montagebauteil montieren. Das Montagebauteil selbst kann mit dem Lichtmodul auch auf einfachere Art und Weise an dem Fahrzeug montiert werden, da der Stecker ein integraler Bestandteil des Lichtmoduls ist und nicht gesondert durch eine Öffnung insbesondere in ein Karosserieteil, wie einer Tür oder dergleichen eingefädelt werden muss.

Des Weiteren kann es vorgesehen sein, dass der Stecker anhand von Haltemitteln auch zur Befestigung des Lichtmoduls an dem Montagebauteil dient. Folglich dient der Stecker nicht nur dem Zweck, das Lichtmodul mit elektrischer Energie zu versorgen, sondern gleichzeitig dient der Stecker auch zur Befestigung des Lichtmoduls an dem Montagebauteil. Die entsprechenden Haltemittel können dabei als Rast- oder Clipsmittel ausgestaltet sein, die mit entsprechenden Gegenhaltemitteln am Montagebauteil zusammenwirken.

Das gesamte Lichtmodul kann ferner mittels einer Rastverbindung einclipsbar am Montagebauteil befestigbar sein. Somit ist das Lichtmodul durch einen besonders einfachen Fertigungsschritt an dem Montagebauteil befestigbar. Optional oder zusätzlich kann das Lichtmodul über eine Schraubverbindung unlösbar mit dem Montagebauteil verbunden werden. Auch ist es denkbar, dass das Lichtmodul an einer Stelle über eine Schweißverbindung, insbesondere eine Punktschweißverbindung, befestigt wird. Auch kann an dem Lichtmodul eine Befestigungslasche angeordnet sein, die zur Befestigung an dem Montagebauteil dient.

Zweckmäßigerweise ist der Stecker an dem Lichtmodul als eine Steckerbuchse ausgestaltet, die mit einem männlichen Steckerteil fahrzeugseitig verbindbar ist. Hierbei kann insbesondere ein Rastverbindungsmittel zur Verbindung der beiden Steckerteile vorgesehen sein. Selbstverständlich kann auch der Stecker an dem Lichtmodul als männliches Steckerteil ausgestaltet sein. Zusätzlich weist der Stecker ein Verpolungsschutz auf, so dass die Steckerbuchse und das männliche Steckerteil in nur einer Stellung ineinander fügbar sind, um einen elektrischen Kontakt zwischen dem Lichtmodul und der fahrzeugseitigen Elektrik herstellen zu können. Gleichzeitig kann dieser Verpolungsschutz integriert mit dem Rastverbindungsmittel sein. Dieses Rastverbindungsmittel kann beispielsweise einen Clips-oder Bajonettverschluss aufweisen.

Des Weiteren ist es denkbar, dass der Stecker des Lichtmoduls an einem Lagerarm eines als Türgriff, insbesondere Türaußengriff, ausgestalteten Montagebauteils anliegt und ein übriger Hauptteil des Lichtmoduls an einem Innebereich einer Handhabe des Türaußengriffes angeordnet ist. Hierzu kann das Gehäuse des Lichtmoduls L-förmig ausgestaltet sein und in eine insbesondere L-förmige Aufnahme im Montagebauteil, welches als Türgriff ausgestaltet sein kann, eingebettet sein. Somit lässt sich der Stecker an eine gut zugängliche Stelle positionieren, um eine einfache Verbindung mit dem fahrzeugseitigen Stecker zu erreichen. Dabei ist es von besonderem Vorteil, dass der Stecker auch bereits durch eine Öffnung im Karosserieteil ragen kann, wenn das Montagebauteil am Fahrzeug montiert wird, um diesen dann mit dem fahrzeugseitigen Gegenstecker zu verbinden.

Ferner hat es sich als zweckmäßig herausgestellt, dass das Gehäuse des Lichtmoduls mit dem Stecker in einem Spritzgussverfahren, insbesondere Kunststoffspritzgussverfahren herstellbar ist. Wie bereits erwähnt kann dadurch das Fertigungsverfahren des Lichtmoduls vereinfacht und kostengünstiger ausgestaltet werden. Außerdem können auf herausragende elektrische Leitungen zwischen dem Lichtmodul und dem Stecker verzichtet werden. Des Weiteren hat es sich als besonders vorteilhaft herausgestellt, dass das Gehäuse des Lichtmoduls lichtdurchlässiges Material aufweist. Bei diesem lichtdurchlässigen Material kann es sich insbesondere um Kunststoff beispielsweise in Form von Polycarbonat (PC) beziehungsweise Makrolon handeln. Somit ist das gesamte Gehäuse lichtdurchlässig ausgestaltet, so dass selbst der Stecker aus diesem lichtdurchlässigen Material bestehen kann. Um das Licht von dem Leuchtmittel in dem Lichtmodul jedoch nicht wahllos austreten zu lassen, kann zumindest eine optische Linse für einen Lichtstrahl des Leuchtmittels vorhanden sein, um diesen Lichtstrahl gegebenenfalls zu bündeln oder zu streuen. Hierbei ist es besonders vorteilhaft, wenn die vorgesehene Linse ebenfalls einteilig zum Gehäuse des Lichtmoduls ausgestaltet ist. Somit lässt sich das Gehäuse mit der Linse und dem Stecker für das Lichtmodul in einem Fertigungsverfahren herstellen. Selbstverständlich können auch mehrere Linsen in dem Lichtmodul angeordnet sein. Bei den Linsen kann es sich selbst um verschiedene Linsenformen, wie zum Beispiel konvexe oder konkave Linsen handeln. Somit kann durch diese Linsen ein Breitstrahl oder ein Richtstrahl erzeugt werden.

Auch kann innerhalb des Lichtmoduls eine halblichtdurchlässige Schicht vorgesehen sein, um den Lichtstrahl vom Leuchtmittel aufzuteilen, nämlich in einen Lichtstrahl, der durch die halblichtdurchlässige Schicht verläuft, und in einen weiteren Lichtstrahl, der an der halblichtdurchlässigen Schicht in eine andere Richtung reflektiert wird. Somit ist es möglich, dass der von nur einem Leuchtmittel ausgestrahlte Lichtstrahl in zumindest zwei unterschiedlichen Richtungen, insbesondere in 60° bis 120° versetzten Richtungen, aus dem Lichtmodul austritt.

Bei der Erfindung kann das Lichtmodul mit genau einem Leuchtmittel ausgestaltet sein. Bei diesem Leuchtmittel kann es sich um eine LED, die insbesondere weißes Licht abstrahlt, handeln. Trotzdem ist es denkbar, dass aus dem Lichtmodul an unterschiedlichen Seiten der Lichtstrahl des Leuchtmittels ausstrahlt. Hierzu können die bereits genannten Linsen genutzt werden, um den Lichtstrahl aufzuteilen, wobei auch reflektierende Flächen zum Einsatz kommen können, um einen Teil des Lichtstrahls abzulenken. Selbstverständlich können auch mehrere Leuchtmittel in dem Lichtmodul angeordnet sein, um eine höhere Leuchtintensität zu erhalten. Auch ist es denkbar, dass das weiße Licht durch eine RGB-LED-Baugruppe erzeugt wird, so dass das rote, das grüne und das blau Licht derart überlappt werden, dass weißes Licht erzeugt wird. Als Leuchtmittel kann eine gängige LED oder eine OLED (organische LED) verwendet werden, die insbesondere großflächig ausgestaltet sein kann. Wie bereits erwähnt können auch mehrere Leuchtmittel in Form von LEDs innerhalb eines Lichtmoduls zum Einsatz kommen. Hierbei können auch unterschiedlich farbig leuchtende Leuchtmittel zum Einsatz kommen. Wie bereits zuvor beschrieben worden ist, kann das Lichtmodul zu einer ersten Seite einen Breitstrahl mit einem breit aufgefächerten Lichtkegel und/oder zu einer zweiten Seite einen Richtstrahl mit einem fokussierten Lichtkegel ausstrahlen. Selbstverständlich können auch zu beiden Seiten Breitstrahle oder Richtstrahle ausgestrahlt werden. Durch die Ausstrahlung des Lichts im Lichtmodul an zwei Seiten kann insbesondere eine Vorfeldbeleuchtung für ein Fahrzeug und/oder eine Muldenbeleuchtung für einen Türgriff oder dergleichen erreichbar sein. Selbstverständlich ist es auch denkbar, dass der Lichtstrahl vom Leuchtmittel auch zu einer dritten Seite aus dem Lichtmodul austritt.

Auch ist es denkbar, dass das Lichtmodul zwei voneinander optisch getrennte Fenster aufweist, aus denen die Lichtstrahlen vom Leuchtmittel, insbesondere ausgerichtet austreten. Durch diese beiden Fenster können die Lichtstrahlen in einem Winkel zwischen 60° bis 120°, bevorzugt zwischen 80° und 100°, ganz bevorzugt ca. 90° zueinander austreten. Die beiden Fenster am Lichtmodul können durch eine Blende am außenliegenden Montagebauteil, insbesondere einem Türaußengriff, erreicht werden. Diese Blende trennt die beiden Fenster optisch voneinander. Gleichzeitig kann die entsprechende Blende am außenliegenden Montagebauteil auch als Haltesteg für das Lichtmodul dienen. Hinter diesem Haltesteg kann das Lichtmodul formschlüssig zur Befestigung am Montagebauteil, insbesondere einem Türaußengriff, angeordnet werden. Durch die beiden Fenster am Lichtmodul ist es möglich, den Lichtstrahl gezielt auf die zu beleuchtenden Bereiche am Fahrzeug auszurichten. Somit kann verhindert werden, dass z. B. ein nachfolgendes Fahrzeug in der Dunkelheit von dem Lichtmodul geblendet wird. Zu diesem Zweck wird der austretende Lichtstrahl von dem Leuchtmodul ausschließlich auf die Griffmulde beim Fahrzeug gerichtet, so dass der Türaußengriff eine Blendung des nachfolgenden Fahrzeugs verhindert. Außerdem kann der Lichtstrahl auch nur auf den Boden des Fahrzeugs fokussiert werden, so dass auch hier eine Blendung des nachfolgenden Verkehrs mit großer Sicherheit vermieden wird.

Des Weiteren kann es erfindungsgemäß vorgesehen sein, dass das Montagebauteil selbst über eine Elektronikeinheit verfügt, die über einen Stecker mit der fahrzeugseitigen Elektronik verbunden wird. Hierbei kann der Stecker des Montagebauteils einteilig mit dem Stecker des Lichtmoduls ausgestaltet sein. Die beiden Stecker können dabei über eine geschlossene Führung oder eine Clipsverbindung oder dergleichen zu einem Kombistecker verbunden sein. Auch ist es denkbar, dass die beiden Stecker eine einstückige Einheit bilden, indem zum Beispiel die elektrischen Leitungen des Montagebauteils in den Stecker des Lichtmoduls führen. Folglich muss bei der Montage des außenliegenden Montagebauteils an dem Fahrzeug nur ein Stecker oder ein Kombistecker mit der fahrzeugseitigen Elektronik verbunden werden. Somit lässt sich der Montageaufwand des Montagebauteils am Fahrzeug weiter vereinfachen.

Ferner ist es denkbar, dass ein Helligkeitssensor vorhanden ist, der zur Ansteuerung des Lichtmoduls, insbesondere des Leuchtmittels, dient, wobei insbesondere der Helligkeitssensor im Lichtmodul integriert ist. Somit lässt sich das Leuchtmittel derart ansteuern, dass es nur ab einer gewissen Dunkelheit eingeschaltet wird und nicht unnötigerweise bei Tage Strom verbraucht. Selbstverständlich kann der Helligkeitssensor auch in dem Montagebauteil oder in der fahrzeugseitigen Elektronik angeordnet sein, um das Leuchtmittel des Lichtmoduls anzusteuern. Bei einem integrierten Helligkeitssensor in dem Lichtmodul ist jedoch der Steuerungsaufwand besonders einfach und es kann auf eine weitere Steuerleitung zur Fahrzeugelektronik verzichtet werden.

Ferner kann es vorgesehen sein, dass das Lichtmodul mit einem Sicherheitssystem, insbesondere einem Zugangsberechtigungssystem, in Signalverbindung steht, welches einen mobilen ID-Geber zur schlüssellosen Aktivierung eines Ver- und Entriegelungsvorganges einer Schließvorrichtung aufweist, wobei über das Sicherheitssystem das Lichtmodul einschaltbar ist. Hierbei kann insbesondere zur Ver- und Entriegelung eine Datenkommunikation zwischen dem ID-Geber und der Schließvorrichtung erfolgen und ein Code ausgetauscht werden, wobei erst nach einer positiven Auswertung des Codes das Sicherheitssystem das Lichtmodul ansteuert bzw. einschaltet. Als Sicherheitssystem kann ein Zugangsberechtigungssystem im Sinne der Erfindung vorgesehen sein. Somit kann verhindert werden, dass das Lichtmodul sich unzulässiger Weise einschaltet und Energie verbraucht, wenn eine unberechtigte Person sich dem Fahrzeug nähert oder einen Zugang zum Fahrzeug verschaffen will.

Das Lichtmodul kann selbst eine Steuerelektronik zur Ansteuerung des Leuchtmittels aufweisen, wobei die Steuerelektronik über den Stecker mit elektrischer Energie versorgt wird. Die gesamte Steuerelektronik kann durch eine Abdichtung in Form eines Deckels und/oder durch eine zusätzliche Vergussmasse im Lichtmodul geschützt angeordnet werden.

Durch die Steuerelektronik lässt sich das Leuchtmittel durch eine Pulsweitenmodulation ansteuern.

Ferner ist die vorliegende Erfindung auch auf ein Fertigungsverfahren für ein außenliegendes Montagebauteil bei einem Fahrzeug gemäß den Merkmalen von Anspruch 15 gerichtet. Hierbei kann insbesondere ein Montagebauteil gemäß den Ansprüchen 1 bis 14 Verwendung finden. Bei dem Fertigungsverfahren ist es erfindungsgemäß vorgesehen, dass die Montage des Lichtmoduls mit dem dazugehörigen Stecker in einem Fertigungsschritt erfolgt. Somit wird nicht zunächst das Lichtmodul an dem Montagebauteil befestigt und anschließend der Stecker sondern diese beiden Schritte finden in genau einem Fertigungsschritt statt. Ebenfalls wird das Gehäuse des Lichtmoduls mit dem Stecker in einem Fertigungsschritt hergestellt.

Weitere Maßnahmen und Vorteile der Erfindung ergeben sich aus den Ansprüchen, der nachfolgenden Beschreibung und den Zeichnungen. Ebenfalls gelten die offenbarten Merkmale aus der erfindungsgemäßen Vorrichtung auch für das erfindungsgemäße Verfahren und umgekehrt. In den Zeichnungen ist die Erfindung in unterschiedlichen Ausführungsbeispielen dargestellt. Dabei können die in den Ansprüchen und in der Beschreibung erwähnten Merkmale jeweils einzeln für sich oder in beliebiger Kombination erfindungswesentlich sein.
- Figur 1: teilweise dreidimensionale Darstellung eines erfindungsgemäßen Montagebauteils in Form eines Türaußengriffes,
- Figur 2: dreidimensionale Draufsicht auf ein Lichtmodul für das Montagebauteil,
- Figur 3: Explosionsansicht auf das Lichtmodul aus Figur 2,
- Figur 4: Rückansicht auf das Lichtmodul aus den Figuren 2 und 3 ohne Deckel,
- Figur 5: teilweise dreidimensionale Ansicht auf das erfindungsgemäße Montagebauteil, jedoch ohne ein montiertes Lichtmodul,
- Figur 6: Ansicht auf ein Fahrzeug mit zwei außenliegenden Montagebauteilen in Form einem Außenspiegel und einem Türaußengriff und
- Figur 7: teilweise dreidimensionale Darstellung eines weiteren erfindungsgemäßen Montagebauteils in Form eines Türaußengriffes mit einem geänderten Lichtmodul.

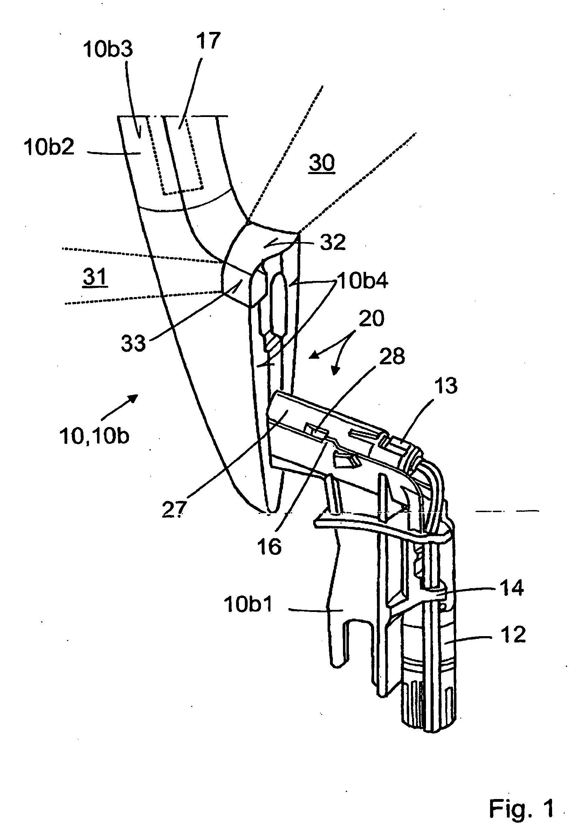

In den Figuren 1 bis 7 sind die gleichen technischen Merkmale aus den jeweiligen Ausführungsbeispielen mit den gleichen Bezugszeichen versehen.

In Figur 1 wird ein erstes Ausführungsbeispiel des erfindungsgemäßen Montagebauteils 10 für ein Fahrzeug 100 dargestellt, welches in der Regel außenliegend am Fahrzeug 100 angeordnet ist. Bei diesem Montagebauteil 10 ist bereits ein Lichtmodul 20 in einer Aufnahme 11 im Montagebauteil 10 angeordnet. Die Montage des Lichtmoduls 20 an dem Montagebauteil 10 erfolgt in der Regel durch ein einfaches Hineindrücken in die Aufnahme 11, wobei Rastverbindungsmittel vorgesehen sind, die das Lichtmodul 20 unlösbar an dem Montagebauteil 10 halten. Diese Rastverbindungsmittel können aus einer Clipsverbindung oder einer geschlossenen Führung bestehen. Im vorliegenden Fall kann auch eine vorgesehene Befestigungslasche 21.2 am Lichtmodul 20 als Rastverbindungsmittel dienen. Zusätzlich kann das Lichtmodul 20 über eine Schraubverbindung oder eine Schweißverbindung mit dem Montagebauteil 10 verbunden werden. Hierfür ist in der Befestigungslasche 21.2 eine Bohrung vorgesehen, durch die eine Verschraubung oder Verschweißung stattfinden kann. Die Befestigungslasche 21.2 kann selbst hinter federnde Vorsprünge, die am Montagebauteil 10 angeordnet sind, fahren, wodurch die bereits erwähnte Rastverbindung 19 erzeugt wird. Zusätzlich dient ein Stecker 27, der einteilig zum Gehäuse 21 des Lichtmoduls 20 ausgestaltet ist, zur Befestigung des Lichtmoduls 20. Hierfür sind an dem Stecker 27 herausragende Haltemittel 28, in Form von Vorsprüngen, angeordnet, die mit entsprechenden Gegenhaltemitteln 16 am außenliegenden Montagebauteil 10 form- und/oder kraftschlüssig zusammenwirken. Ebenfalls können die Haltemittel 28 und Gegenhaltemittel 16 über Verrastungsmittel verfügen.

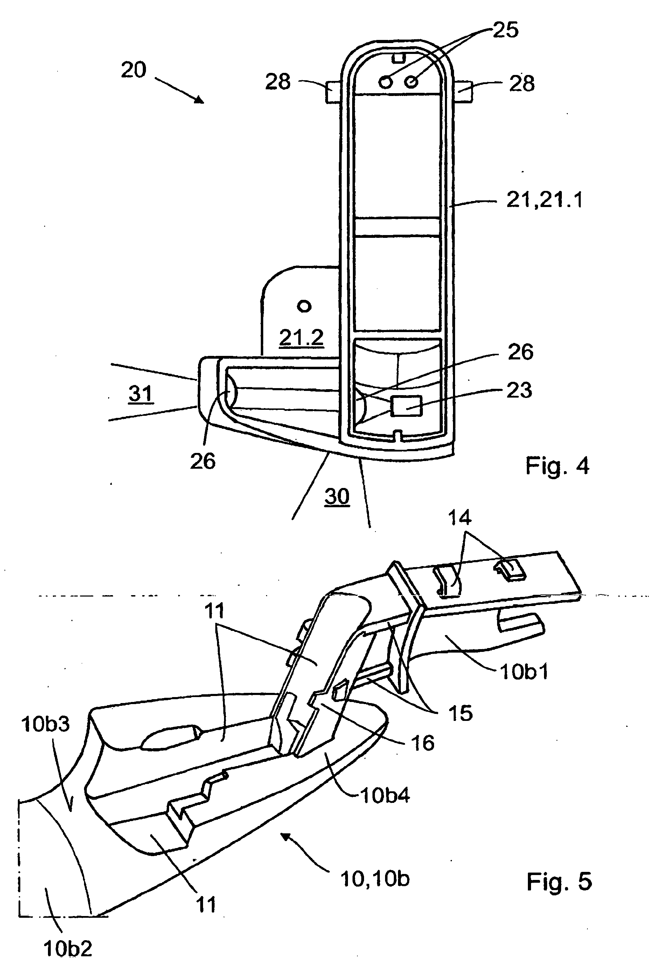

Damit sich das Lichtmodul 20 formschön und unauffällig im außenliegenden Montagebauteil 10 integrieren lässt, ist die rückseitige Aufnahme 11 im Montagebauteil 10 vorgesehen (s. Fig. 5). In diese Aufnahme 11 wird das L-förmig ausgestaltete Lichtmodul 20 derart eingebettet, dass es eine bündige Anlagefläche 10b4 mit dem Montagebauteil 10 bildet. Diese Anlagefläche 10b4 kann dazu dienen, das Montagebauteil 10 mit dem Fahrzeug 100 zu befestigen. Zwischen der Anlagefläche 10b4 und einem Karosseriebauteil des Fahrzeugs 100 kann eine Dichtung angeordnet sein, so dass eine ebenfalls vorhandene Öffnung im Karosserieteil abgedichtet werden kann.

Wie aus der Figur 1 weiter ersichtlich ist, weist das außenliegende Montagebauteil 10 selbst eine Elektronikeinheit 17 auf, die zum Beispiel für ein Sicherheitssystem des Fahrzeuges 100 arbeitet. Diese Elektronikeinheit 17 ist in Figur 1 gestrichelt angedeutet und wird über einen weiteren Stecker 12 mit der fahrzeugseitigen Elektronik verbunden. Dieser Stecker 12 ist an einem Lagerarm 10.1 des Türaußengriffes 10b befestigt. Der Lagerarm 10.1 ragt durch eine Öffnung im Karosserieteil des Bauteils 100 und dient dazu, den Türgriff 10b beweglich zu lagern. Zusätzlich ist ein Gegenstecker 13 vorgesehen, der mit dem Stecker 27 des Lichtmoduls 20 verbunden ist, um dieses mit elektrischer Energie zu versorgen. Der Stecker 27 ist hierbei als Steckerbuchse 27.1 ausgestaltet und nimmt den Gegenstecker 13 auf. Bei einer besonderen Variante, die jedoch nicht in Figur 1 dargestellt ist, kann der Stecker 12 und der Stecker 27 einteilig ausgestaltet sein, so dass sowohl die Elektronikeinheit 17 des Montagebauteils 10 als auch eine Steuerelektronik 24 beziehungsweise ein Leuchtmittel 23 mit elektrischer Energie des Lichtmoduls 20 versorgt werden kann. Damit die Anschlussleitung vom Stecker 13 nicht lose rumhängt, sind zusätzlich die gegeneinander versetzten Befestigungshaken 14 am Lagerarm 10b1 des Türgriffs 10b vorgesehen (s. Fig. 5).

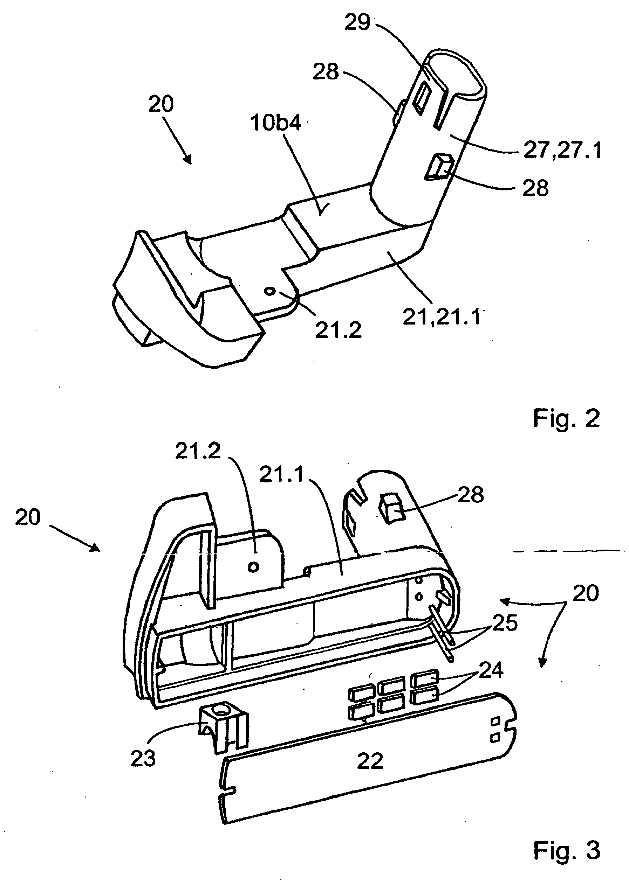

In der Figur 2 ist das Lichtmodul 20 ohne das Montagebauteil 10 dargestellt. Wie gut zu erkennen ist, weist das Gehäuse 21 einen Hauptteil 21.1 und den Stecker 27 auf. Dabei ist der Stecker 27 senkrecht zum Hauptteil 21.1 angeordnet, so dass das gesamte Gehäuse 21 des Lichtmoduls 20 eine L-förmige Ausgestaltung annimmt. Damit der Stecker 27 eine feste Verbindung mit seinem Gegenstecker 23 eingehen kann, ist ein Rastverbindungsmittel 29 vorgesehen, welches laschenartig am oberen Steckerrandende angeordnet ist. Das laschenartige Rastverbindungsmittel 29 entsteht durch zwei Einschnitte im Öffnungsrand des Steckers 27 und steht somit federnd gelagert hervor. Gleichzeitig dient das Rastverbindungsmittel 29 als Verpolungsschutz, da es eine Ausnehmung beziehungsweise Öffnung aufweist, in die ein Vorsprung des Steckers 13 hineinragt, wenn der Stecker 27 mit dem Gegenstecker 27 verbunden ist. Durch den Vorsprung am Stecker 13 lässt sich dieser nicht verkehrt herum in den Stecker 27 einführen. Gleichzeitig sorgt dieser Vorsprung aber auch für einen Formschluss, wenn der Vorsprung in der Öffnung am Rastverbindungsmittel 29 einfährt.

Das gesamte Gehäuse 21 des Lichtmoduls 10 wird vorzugsweise in einem Kunststoffspritzgussverfahren hergestellt, wobei insbesondere ein lichtdurchlässiger Kunststoff in Form von Polycarbonat (PC) verwendet werden kann. Folglich ist auch der Stecker 27 aus dem lichtdurchlässigen Material ausgestaltet. Selbstverständlich können die transparenten Oberflächen des Lichtmoduls 20, aus denen keine Lichtstrahlen vom Leuchtmittel 23 austreten sollen, lackiert werden. Die Lichtstrahlen von dem Leuchtmittel 23 treten in Form von Lichtkegeln 30, 31 aus dem Lichtmodul 20 aus. Dabei tritt aus einer ersten Seite 32 ein aufgefächerter Lichtstrahl 30, der für eine Muldenbeleuchtung für einen Innenbereich 10b3 des Türgriffs 10b dient. Aus der zweiten Seite 33 strahlt hingegen der Lichtkegel 31 aus, der als Vorfeldbeleuchtung für das Fahrzeug 100 dient. Aus der Figur 4 geht der Strahlengang vom Leuchtmittel 23 zu den Lichtkegeln 30, 31 im Gehäuse 21 des Lichtmoduls 20 hervor.

Die Figur 3 zeigt eine Explosionsansicht des Lichtmoduls 20 wobei auch die entsprechende Steuerelektronik 24 und das Leuchtmittel 23 dargestellt sind. Die Steuerelektronik 24 ist auf einer Leiterplatte 22 angeordnet, die auch das Leuchtmittel 23 aufnimmt. Bevorzugt kommt hierbei die SMD-Technologie zum Einsatz. Die Leiterplatte 22 kann gleichzeitig als Deckel 22 für das Gehäuse 21 des Lichtmoduls 20 dienen, wobei die Leiterplatte 22 über zwei Verbindungsstäbe 25, die am Gehäuse 21 angespritzt sind, mit dem Gehäuse 21 insbesondere über Schweißverbindungen verbunden werden kann. Damit die gesamte Elektronik des Lichtmoduls 20 gegen Feuchtigkeit und andere Umwelteinflüsse geschützt ist, kann es mit einer Vergussmasse abgedichtet werden. Die Leiterplatte 22 kann zusätzlich mit Dichtmitteln am Gehäuse 21 dichtend zusammenwirken. Durch die zusätzlich aufgebrachte Vergussmasse ist ein sicherer Schutz vor Feuchtigkeit möglich.

In der Figur 4 ist eine Rückansicht des Lichtmoduls 20 dargestellt, wobei das Leuchtmittel 23 eingezeichnet ist, um den Strahlengang des Lichts im Lichtmodul 20 anzudeuten. Wie zu erkennen ist, kommt in dem vorliegenden Fall nur genau ein Leuchtmittel 23 zum Einsatz, welches am unteren Ende (bezogen auf Figur 4) im Hauptteil 21.1 des Gehäuses 21 angeordnet ist. Dieses Leuchtmittel 23 strahlt nur zu einer Seite ab, nämlich nach links, wobei der ausgestrahlte Lichtstrahl auf eine erste Linse 26 trifft, die konkav ausgestaltet ist. Hinter dieser Linse 26 befindet sich eine reflektierende Schicht, die den Lichtstrahl für die beiden Lichtkegel 30 und 31 aufteilt. Die Lichtkegel 30, 31 können pyramidenstumpfförmig oder kegelstumpfförmig ausgestaltet sein. Der Lichtkegel 30 tritt - um ca. 90 ° abgelenkt - aufgefächert aus der ersten Seite 32 aus dem Gehäuse 21 aus. Ein nicht reflektierter Teil des Lichtstrahls hinter der ersten Linse 26 trifft mehr oder weniger unabgelenkt weiter auf eine zweite Linse 26 und wird dort teilweise fokussiert, um als Lichtkegel 31 aus der zweiten Seite 33 auszutreten. Die beiden Linsen 26 sind im vorliegenden Ausführungsbeispiel materialeinheitlich und einteilig zum Gehäuse 21 des Lichtmoduls 20 ausgestaltet. Somit ist eine besonders einfache und kostengünstige Fertigung des Lichtmoduls 20 möglich, da zusätzliche Linsen nicht hergestellt und montiert werden müssen.

In der Figur 5 ist die ebenfalls L-förmige Aufnahme 11 am Montagebauteil 10, welches als Türgriff 10b ausgestaltet ist, dargestellt. Dabei wird der Hauptteil 21.1 des Lichtmoduls 20 in einer Öffnung der Anlagefläche 10.4 für die Aufnahme 11 eingebettet, die sich auf der Rückseite einer Handhabe 10b2 vom Türgriff 10b befindet. Der senkrecht vom Hauptteil 21.1 herausragende Stecker 27 wird hingegen in dem Lagerarm 10b1 beziehungsweise dem entsprechenden Aufnahmebereich 11 angeordnet. Dabei hinterfährt das Haltemittel 28 vom Stecker 27 formschlüssig das als Vorsprung ausgestaltete Gegenhaltemittel 16 des Montagebauteils 10. Ebenfalls sind zwei Befestigungshaken 14 an der Rückseite des Lagerarms 10b1 angeordnet, mit deren Hilfe ein fahrzeugseitiges Kabel für den Gegenstecker 13 befestigt werden kann. Zur Erhöhung der Stabilität des Lagerarms 10b1 sind diverse Verstärkungsrippen 15 vorgesehen.

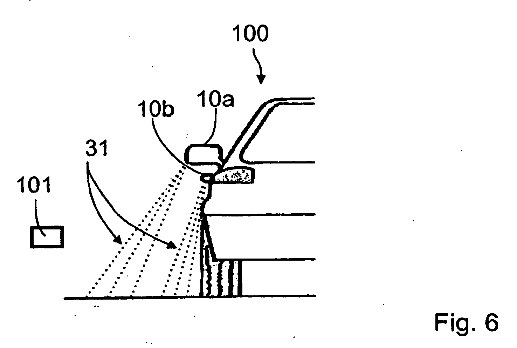

In der Figur 6 ist beispielsweise ein Fahrzeug 100 als KFZ dargestellt, bei dem das außenliegende Montagebauteil 10 einerseits als Außenspiegel 10a und andererseits als Türgriff 10b ausgestaltet ist und eine Vorfeldbeleuchtung 31 erzeugt, wobei ebenfalls eine Muldenbeleuchtung 30 denkbar ist. Zur Ansteuerung des Lichtmoduls 20 kann ein Signal von einem ID-Geber 101 für ein Sicherheitssystem, insbesondere in Form eines Zugangsberechtigungssystems, von dem Fahrzeug 100 dienen. Nähert sich eine zugangsberechtigte Person mit dem entsprechenden ID-Geber 101 dem Fahrzeug 100, so kann das Lichtmodul 20 angesteuert werden, welches eine Vorfeldbeleuchtung 31 vornimmt, wenn es entsprechend dunkel ist. Selbstverständlich kann das Lichtmodul 20 auch über andere fahrzeugseitige Systeme angesteuert werden.

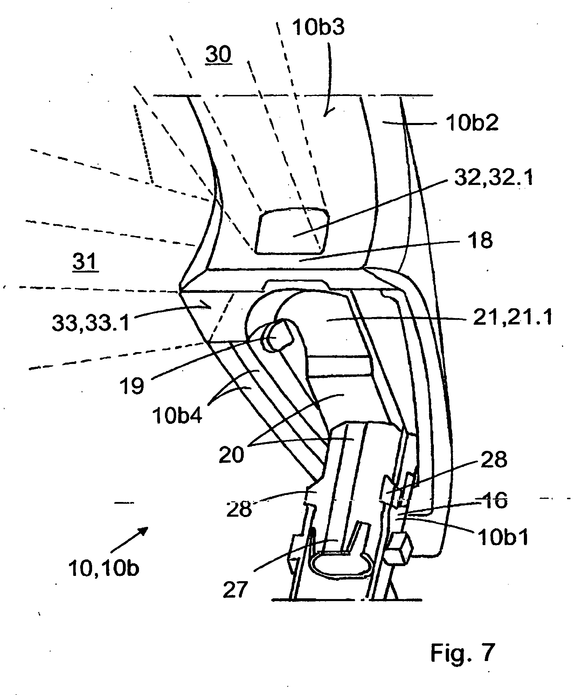

In der weiteren Figur 7 ist ein vergleichbares Ausführungsbeispiel zur Figur 1 des erfindungsgemäßen Montagebauteils 10 für ein Fahrzeug 100 dargestellt. Bei diesem Ausführungsbeispiel in Figur 7 ist am Türaußengriff 10b eine Blende 18 für das entsprechende Lichtmodul 20 vorgesehen. Diese Blende 18 dient im vorliegenden Beispiel gleichzeitig als zusätzlicher Haltesteg für das Lichtmodul 20, um dieses mechanisch an dem Türaußengriff 10b zu fixieren. Dabei ragt ein erstes, insbesondere quaderförmiges Fenster 32.1 der ersten Seite 32 vom Lichtmodul 20 durch eine Durchbruchsöffnung im Türaußengriff 10b, die durch die Blende 18 gebildet wird. Durch dieses erste Fenster 32.1 scheint der Lichtstrahl vom Leuchtmittel 23 als Lichtkegel 30 zur Muldenbeleuchtung. In der zweiten Seite 33 des Lichtmoduls 20 ist ein zweites Fenster 33.1 vorgesehen, durch welches ebenfalls der Lichtstrahl vom Leuchtmittel 23 austritt und als Lichtkegel 31 (zur Vorfeldbeleuchtung bzw. Bodenbeleuchtung vor der Fahrzeugtür) erscheint. Wie gut zu erkennen ist, sind durch die beiden Fenster 32.1 und 33.1 die austretenden Lichtstrahlen eindeutig auf den jeweils auszuleuchtenden Bereich gerichtet. So dient das erste Fenster 32.1 ausschließlich dazu, die Griffmulde vom Türaußengriff 10b zu beleuchten. Das zweite Fenster 33.1 dient zur Bodenbeleuchtung vor der Fahrzeugtür. Durch die zusätzliche Blende 18 wird erreicht, dass die Lichtstrahlen vom Leuchtmodul 20 nicht unterhalb oder oberhalb des Türaußengriffs 10b unkontrolliert ausgestrahlt werden, wodurch ein nachfolgendes Fahrzeug geblendet werden könnte.

Das gesamte Lichtmodul 20 wird in dem Ausführungsbeispiel aus Figur 7 über das dargestellte Rastelement 19 am Türaußengriff 10b unverlierbar fixiert. Durch die vorhandene Rastverbindung 19 ist jedoch das Lichtmodul 20 reversibel austauschbar am Türaußengriff 10b bzw. dem entsprechenden Montagebauteil 10 angeordnet. Ansonsten entspricht das Ausführungsbeispiel aus Figur 7 mit den weiteren technischen Merkmalen dem Ausführungsbeispiel aus Figur 1.

Durch das erste Fenster 32.1 kann erreicht werden, dass der austretende Lichtstrahl (Lichtkegel 30) sich nicht mehr als in einem Winkel von 15°, insbesondere 10°, gemessen von der Fahrzeugkarosserie nach hinten ausbreitet, um somit eine Blendung des nachfolgenden Verkehrs sicher zu vermeiden. Auch das zweite Fenster 33.1 kann derart auf den Boden des Fahrzeugs gerichtet sein, dass der Lichtkegel 31 ebenfalls nicht mehr als 15° von der Fahrzeugkarosserie raus ragt bzw. abstrahlt.

Ebenfalls sei erwähnt, dass die Blende 18 zur optischen Trennung der beiden Fenster 32.1 und 33.1 auch direkt an dem Lichtmodul 20 angeordnet werden kann. Diese kann z. B. aufgespritzt, aufgeklebt oder durch ein Druck- oder Lackierprozess aufgebracht werden. In diesem Fall geht jedoch keine mechanische Haltewirkung von der Blende 18 zur Befestigung des Lichtmoduls 20 am außenliegenden Montagebauteil 10 aus.

### Bezugszeichenliste

- 10: Außenliegendes Montagebauteil
- 10a: Außenspiegel
- 10b: Türaußengriff
- 10b1: Lagerarm
- 10b2: Handhabe
- 10b3: Innenbereich
- 10b4: Anlagefläche
- 11: Aufnahme für 20
- 12: Stecker von 10
- 13: Gegenstecker von 100
- 14: Befestigungshaken
- 15: Verstärkungsrippen
- 16: Gegenhaltemittel
- 17: Elektronikeinheit
- 18: Blende / Haltesteg
- 19: Rastverbindung
- 20: Lichtmodul
- 21: Gehäuse
- 21.1: Hauptteil
- 21.2: Befestigungslasche
- 22: Deckel / Leiterplatte
- 23: Leuchtmittel
- 24: Steuerelektronik
- 25: Verbindungsstäbe
- 26: Linse
- 27: Stecker
- 27.1: Steckerbuchse
- 28: Haltemittel
- 29: Verpolungsschutz / Rastverbindungsmittel
- 30: Lichtkegel, insbesondere aufgefächert für Muldenbeleuchtung
- 31: Lichtkegel, insbesondere fokussiert für Vorfeldbeleuchtung
- 32: erste Seite
- 32.1: erstes Fenster
- 33: zweite Seite
- 33.1: zweites Fenster
- 100: Fahrzeug, insbesondere KFZ oder Motorrad
- 101: ID-Geber für Sicherheitssystem, insbesondere Zugangsberechtigungssystem

## Patentansprüche

1. Außenliegendes Montagebauteil (10) für ein Fahrzeug (100), insbesondere in Form eines Außenspiegels (10a), Türgriffs (10b), Türschweller, Schürze oder dergleichen, mit
einem Lichtmodul (20), welches zumindest ein nach außen, sichtbares Licht (30, 31) emittierendes Leuchtmittel aufweist, und
einem Stecker (27), der das Lichtmodul (20), insbesondere das Leuchtmittel (23), mit elektrischer Energie versorgt, wobei das Lichtmodul (20) ein separates Bauteil zum Montagebauteil (10) darstellt, welches an dem Montagebauteil (10) befestigbar ist und der Stecker (27) einteilig mit dem Lichtmodul (20), insbesondere dem Gehäuse (21) des Lichtmoduls (20), ausgestaltet ist,
**dadurch gekennzeichnet,**
**dass** der Stecker (27) ein integraler Bestandteil des Lichtmoduls (20) ist.

2. Außenliegendes Montagebauteil (10) nach Anspruch 1,
**dadurch gekennzeichnet,**
**dass** der Stecker (27) anhand von Haltemitteln (28) auch zur Befestigung des Lichtmoduls (20) an dem Montagebauteil (10) dient.

3. Außenliegendes Montagebauteil (10) nach Anspruch 1 oder 2,
**dadurch gekennzeichnet,**
**dass** das Lichtmodul (20) mittels zumindest einer Rastverbindung (21.2) einclipsbar am Montagebauteil (10) befestigbar ist.

4. Außenliegendes Montagebauteil (10) nach einem der vorherigen Ansprüche,
**dadurch gekennzeichnet,**
**dass** der Stecker (27) eine Steckerbuchse (27.1) aufweist, die mit einem männlichen Steckerteil (13) fahrzeugseitig verbindbar ist, wobei insbesondere Rastverbindungsmittel (29) zur Verbindung vorgesehen sind.

5. Außenliegendes Montagebauteil (10) nach einem der vorherigen Ansprüche,
**dadurch gekennzeichnet,**
**dass** der Stecker (27) an einem Lagerarm (10b1) eines als Türaußengriff (10b) ausgestalteten Montagebauteils (10) anliegt und ein übriger Hauptteil (21.1) des Lichtmoduls (20) an einem Innenbereich (10b3) einer Handhabe (10b2) des Türaußengriffs (10b) angeordnet ist.

6. Außenliegendes Montagebauteil (10) nach einem der vorherigen Ansprüche,
**dadurch gekennzeichnet,**
**dass** ein Gehäuse (21) des Lichtmoduls (20) mit dem Stecker (27) in einem Spritzgussverfahren herstellbar ist.

7. Außenliegendes Montagebauteil (10) nach einem der vorherigen Ansprüche,
**dadurch gekennzeichnet,**
**dass** das Gehäuse (21) des Lichtmoduls (20) lichtdurchlässiges Material aufweist, insbesondere Kunststoff beispielsweise in Form von Polycarbonat (PC) bzw. Makrolon.

8. Außenliegendes Montagebauteil (10) nach einem der vorherigen Ansprüche,
**dadurch gekennzeichnet,**
**dass** das Lichtmodul (20) zumindest eine optische Linse (26) für einen Lichtstrahl (30, 31) des Leuchtmittels (23) aufweist, um diesen zu bündeln oder zu streuen, wobei insbesondere
die Linse (26) einteilig zum Gehäuse (21) des Lichtmoduls (20) ausgestaltet ist.

9. Außenliegendes Montagebauteil (10) nach einem der vorherigen Ansprüche,
**dadurch gekennzeichnet,**
**dass** das Lichtmodul (20) zu einer ersten Seite (32) einen Breitstrahl mit einem breit aufgefächerten Lichtkegel (30) und/oder zu einer zweiten Seite (33) einen Richtstrahl mit einem fokussierten Lichtkegel (31) ausstrahlt, wobei insbesondere eine Vorfeldbeleuchtung (31) und/oder eine Muldenbeleuchtung (30) erreichbar ist.

10. Außenliegendes Montagebauteil (10) nach einem der vorherigen Ansprüche,
**dadurch gekennzeichnet,**
**dass** das Montagebauteil (10) über eine eigene Elektronikeinheit (17) verfügt, die mit einem weiteren Stecker (12) ausgestaltet ist und der Stecker (27) des Lichtmoduls (20) mit dem Stecker (12) des Montagebauteils (10) einteilig ausgestaltet ist.

11. Außenliegendes Montagebauteil (10) nach einem der vorherigen Ansprüche,
**dadurch gekennzeichnet,**
**dass** das Gehäuse (21) des Lichtmoduls (20) L-förmig ausgestaltet ist, und insbesondere in eine L-förmige Aufnahme (11) im Montagebauteil (10) eingebettet ist.

12. Außenliegendes Montagebauteil (10) nach einem der vorherigen Ansprüche,
**dadurch gekennzeichnet,**
**dass** ein Helligkeitssensor vorhanden ist, der zur Ansteuerung des Lichtmoduls (20), insbesondere des Leuchmittels (23), dient, wobei insbesondere der Helligkeitssensor im Lichtmodul (20) integriert ist.

13. Außenliegendes Montagebauteil (10) nach einem der vorherigen Ansprüche,
**dadurch gekennzeichnet,**
**dass** das Lichtmodul (20) mit einem Sicherheitssystem in Signalverbindung steht, das einen mobilen ID-Geber (101) zur schlüssellosen Aktivierung eines Ver- und Entriegelungsvorganges einer Schließvorrichtung aufweist, wobei über das Sicherheitssystem das Lichtmodul (20) einschaltbar ist, wobei
insbesondere zur Ver- und Entriegelung eine Datenkommunikation zwischen dem ID-Geber (101) und der Schließvorrichtung erfolgt und ein Code ausgetauscht wird, wobei erst nach einer positiven Auswertung des Codes das Sicherheitssystem das Lichtmodul (20) ansteuert.

14. Außenliegendes Montagebauteil (10) nach einem der vorherigen Ansprüche,
**dadurch gekennzeichnet,**
**dass** eine Steuerelektronik (24) im Lichtmodul (20) zur Ansteuerung des Leuchtmittels (23) vorgesehen ist, wobei die Steuerelektronik (24) über den Stecker (27) mit elektrischer Energie versorgt wird, und die Steuerelektronik (24) durch eine Abdichtung und/oder Vergußmasse geschützt im Lichtmodul (20) angeordnet ist.

15. Fertigungsverfahren für ein außenliegendes Montagebauteil (10), nach einem der vorherigen Ansprüche, mit einem Lichtmodul (20), welches über einen Stecker (27) mit elektrischer Energie versorgt wird,
**dadurch gekennzeichnet,**
**dass** die Montage des Lichtmoduls (20) mit dem dazugehörenden Stecker (27) in einem Fertigungsschritt erfolgt.

## Claims

1. An external assembly component (10) for a vehicle (100), in particular in the form of an external mirror (10a), door handle (10b), door sill, skirt or suchlike, with
a light module (20), which has at least one lamp emitting visible light (30, 31) toward the exterior, and
a plug (27) which supplies the light module (20), in particular the lamp (23), with electrical energy, wherein the light module (20) represents a separate component to the assembly component (10), which is able to be fastened to the assembly component (10) and the plug (27) is configured in one piece with the light module (20), in particular the housing (21) of the light module (20),
**characterized in that**
the plug (27) is an integral component part of the light module (20).

2. The external assembly component (10) according to Claim 1,
**characterized in that**
the plug (27), with the aid of holding means (28), also serves for fastening the light module (20) on the assembly component (10).

3. The external assembly component (10) according to Claim 1 or 2,
**characterized in that**
the light module (20) is able to be clipped in on the assembly component (10) by means of at least a detent connection (21.2).

4. The external assembly component (10) according to one of the preceding claims,
**characterized in that**
the plug (27) has a socket (27.1) which is able to be connected with a male plug part (13) on the vehicle side, wherein in particular detent connection means (29) are provided for the connection.

5. The external assembly component (10) according to one of the preceding claims,
**characterized in that**
the plug (27) lies against a bearing arm (10b1) of an assembly component (10) equipped as an exterior door handle (10b), and a remaining main part (21.1) of the light module (20) is arranged on an inner region (10b3) of a handle (10b2) of the exterior door handle (10b).

6. The external assembly component (10) according to one of the preceding claims,
**characterized in that**
a housing (21) of the light module (20) together with the plug (27) is able to be produced in an injection moulding process.

7. The external assembly component (10) according to one of the preceding claims,
**characterized in that**
the housing (21) of the light module (20) has translucent material, in particular plastic, for example in the form of polycarbonate (PC) or respectively macrolon.

8. The external assembly component (10) according to one of the preceding claims,
**characterized in that**
the light module (20) has at least one optical lens (26) for a light beam (30, 31) of the lamp (23), in order to focus or scatter this, wherein in particular the lens (26) is configured in one piece to the housing (21) of the light module (20).

9. The external assembly component (10) according to one of the preceding claims,
**characterized in that**
the light module (20) emits to a first side (32) a wide beam with a widely fanned-out light cone (30) and/or to a second side (33) a directional beam with a focussed light cone (31), wherein in particular a forefield illumination (31) and/or a recess illumination (30) is able to be achieved.

10. The external assembly component (10) according to one of the preceding claims,
**characterized in that**
the assembly component (10) has its own electronic unit (17), which is configured with a further plug (12), and the plug (27) of the light module (20) is configured in one piece with the plug (12) of the assembly component (10).

11. The external assembly component (10) according to one of the preceding claims,
**characterized in that**
the housing (21) of the light module (20) is configured in an L-shape, and in particular is embedded into an L-shaped mount (11) in the assembly component (10).

12. The external assembly component (10) according to one of the preceding claims,
**characterized in that**
a brightness sensor is present, which serves for activation of the light module (20), in particular of the lamp (23), wherein in particular the brightness sensor is integrated in the light module (20).

13. The external assembly component (10) according to one of the preceding claims,
**characterized in that**
the light module (20) is in signal connection with a safety system, which has a mobile ID transmitter (101) for the keyless activation of a locking and unlocking procedure of a locking device, wherein the light module (20) is able to be switched on via the security system, wherein
in particular for the locking and unlocking, a data communication takes place between the ID transmitter (101) and the locking device, and a code is exchanged, wherein the security system only activates the light module (20) after a positive evaluation of the code.

14. The external assembly component (10) according to one of the preceding claims,
**characterized in that**
an electronic control system (24) is provided in the light module (20) for the activation of the lamp (23), wherein the electronic control system (24) is supplied with electrical energy via the plug (27), and the electronic control system (24), protected by a seal and/or sealing compound, is arranged in the light module (20).

15. A manufacturing method for an external assembly component (10), according to one of the preceding claims, with a light module (20), which is supplied with electrical energy via a plug (27),
**characterized in that**
the assembly of the light module (20) with the associated plug (27) takes place in one manufacturing step.

## Revendications

1. Composant de montage extérieur (10) pour un véhicule (100), en particulier sous la forme d'un rétroviseur extérieur (10a), d'une poignée de porte (10b), d'un seuil de porte, d'une jupe ou analogue, comportant un module lumineux (20) qui présente au moins une source lumineuse émettant vers l'extérieur une lumière visible (30, 31), et
un connecteur (27) qui alimente le module lumineux (20), en particulier la source lumineuse (23), en énergie électrique, le module lumineux (20) constituant un composant séparé du composant de montage (10), qui peut être fixé au composant de montage (10), et le connecteur (27) étant formé d'une seule pièce avec le module lumineux (20), en particulier le boîtier (21) du module lumineux (20),
**caractérisé en ce**
**que** le connecteur (27) fait partie intégrante du module lumineux (20).

2. Composant de montage extérieur (10) selon la revendication 1,
**caractérisé en ce**
**que** le connecteur (27) sert aussi, à l'aide de moyens de retenue (28), à la fixation du module lumineux (20) au composant de montage (10).

3. Composant de montage extérieur (10) selon la revendication 1 ou 2,
**caractérisé en ce**
**que** le module lumineux (20) peut être fixé au composant de montage (10) de façon encliquetable au moyen d'au moins une liaison par encliquetage (21.2).

4. Composant de montage extérieur (10) selon l'une des revendications précédentes,
**caractérisé en ce**
**que** le connecteur (27) présente une partie de connecteur femelle (27.1) qui peut être reliée à une partie de connecteur mâle (13) côté véhicule, des moyens de liaison par encliquetage (29) étant en particulier prévus pour la liaison.

5. Composant de montage extérieur (10) selon l'une des revendications précédentes,
**caractérisé en ce**
**que** le connecteur (27) repose sur un bras de palier (10b1) d'un composant de montage (10) réalisé sous la forme d'une poignée extérieure de porte (10b) et une autre partie principale (21.1) du module lumineux (20) est disposée sur une zone intérieure (10b3) d'une manette (10b2) de la poignée extérieure de porte (10b).

6. Composant de montage extérieur (10) selon l'une des revendications précédentes,
**caractérisé en ce**
**qu'**un boîtier (21) du module lumineux (20) peut être fabriqué avec le connecteur (27) en une opération de moulage par injection.

7. Composant de montage extérieur (10) selon l'une des revendications précédentes,
**caractérisé en ce**
**que** le boîtier (21) du module lumineux (20) présente un matériau translucide, en particulier une matière plastique par exemple sous forme de polycarbonate (PC) ou de Makrolon.

8. Composant de montage extérieur (10) selon l'une des revendications précédentes,
**caractérisé en ce**
**que** le module lumineux (20) présente au moins une lentille optique (26) pour un faisceau lumineux (30, 31) de la source lumineuse (23), pour concentrer ou disperser celui-ci, la lentille (26) étant en particulier formée d'une seule pièce avec le boîtier (21) du module lumineux (20).

9. Composant de montage extérieur (10) selon l'une des revendications précédentes,
**caractérisé en ce**
**que** le module lumineux (20) émet vers un premier côté (32) un faisceau large avec un cône lumineux largement ouvert (30) et/ou vers un deuxième côté (33) un faisceau dirigé avec un cône lumineux focalisé (31), ce permet d'obtenir en particulier un éclairage de proximité (31) et/ou un éclairage de creux de poignée (30).

10. Composant de montage extérieur (10) selon l'une des revendications précédentes,
**caractérisé en ce**
**que** le composant de montage (10) dispose d'une unité électronique (17) propre qui est formée avec un autre connecteur (12) et le connecteur (27) du module lumineux (20) est formé d'une seule pièce avec le connecteur (12) du composant de montage (10).

11. Composant de montage extérieur (10) selon l'une des revendications précédentes,
**caractérisé en ce**
**que** le boîtier (21) du module lumineux (20) est réalisé en forme de L et encastré en particulier dans un logement en forme de L (11) dans le composant de montage (10).

12. Composant de montage extérieur (10) selon l'une des revendications précédentes,
**caractérisé en ce**
**qu'**un capteur de luminosité est présent, qui sert à la commande du module lumineux (20), en particulier de la source lumineuse (23), le capteur de luminosité étant en particulier intégré dans le module lumineux (20).

13. Composant de montage extérieur (10) selon l'une des revendications précédentes,
**caractérisé en ce**
**que** le module lumineux (20) est en liaison de signalisation avec un système de sécurité qui présente un émetteur d'identification mobile (101) pour l'activation sans clé d'une opération de verrouillage et de déverrouillage d'un dispositif de fermeture, le module lumineux (20) pouvant être activé par le système de sécurité,
une communication de données ayant lieu entre l'émetteur d'identification mobile (101) et le dispositif de fermeture en particulier pour le verrouillage et de déverrouillage et un code étant échangé à cette occasion, le système de sécurité n'activant le module lumineux (20) qu'après une évaluation positive du code.

14. Composant de montage extérieur (10) selon l'une des revendications précédentes,
**caractérisé en ce**
**qu'**une électronique de commande (24) est prévue dans le module lumineux (20) pour la commande de la source lumineuse (23), l'électronique de commande (24) étant alimentée en énergie électrique par l'intermédiaire du connecteur (27) et l'électronique de commande (24) étant disposée dans le module lumineux (20) de manière protégée par un moyen d'étanchéité et/ou une masse de scellement.

15. Procédé de fabrication d'un composant de montage extérieur (10) selon l'une des revendications précédentes, comportant un module lumineux (20) qui est alimenté en énergie électrique par l'intermédiaire d'un connecteur (27),
**caractérisé en ce**
**que** le montage du module lumineux (20) avec le connecteur (27) qui en fait partie s'effectue en une étape de fabrication.
